# EUROPEAN PATENT SPECIFICATION

(11) **EP 2 180 091 B1**
(45) Date of publication and mention of the grant of the patent: **12.09.2012**
(21) Application number: 09013240.8
(22) Date of filing: 20.10.2009
(51) Int. Cl.: D02G 3/32, D02G 3/44, A41D 13/12, A61B 5/0408

(54) **Pressure-sensitive conductive yarn and biological information-measuring garment**
Druckempfindliches leitfähiges Garn und Kleidungsstück zur Messung biologischer Daten
Fil conducteur sensible à la pression et vêtement de mesure d'informations biologiques

(30) Priority: 24.10.2008 JP 2008274641
(43) Date of publication of application: 28.04.2010
(73) Proprietor: The Ritsumeikan Trust, Kyoto 604-8520 (JP); OKAMOTO CORPORATION, Kitakatsuragi-gun, Nara, 6358550 (JP)
(72) Inventor: Oda, Syunsuke, Nojihigashi Kusatsu-shi Shiga 525-8577 (JP); Araki, Takahiro, Kitakatsuragi-gun Nara 635-8550 (JP); Fujita, Emi, Kitakatsuragi-gun Nara 635-8550 (JP); Makikawa, Masaaki, Nojihigashi Kusatsu-shi Shiga 525-8577 (JP)
(74) Representative: Müller-Boré & Partner Patentanwälte

(56) References cited:
- WO-A1-2004/097089
- DE-A1-102006 058 765
- JP-A- 2 027 610
- JP-A- 10 001 851
- JP-A- 2000 239 932
- JP-A- 2003 020 538
- US-A1- 2006 211 934
- US-A1- 2008 228 097

## Description

### BACKGROUND OF THE INVENTION

### (1) Field of the Invention

The present invention relates to a pressure-sensitive conductive yarn and a garment for measuring biological information.

### (2) Description of the Related Art

Biological information, e.g., respiration, heart rate, etc., is an important index for determining health conditions; however, known biological signal measurements require application of electrodes using gel, the wrapping of respiration bands, etc., causing great discomfort to the body.

To eliminate such discomfort and facilitate daily health management, some attempts have been made to allow for the detection of biological information in a natural state by applying electrodes to a garment. For example, Patent Document 1 discloses providing electrodes formed of a conductive yarn on a garment to detect pulse signals.
Patent Document 1: Japanese Unexamined Patent Publication No. 2005-525477 (WO-A-03094717)

US-A-2006/211934 discloses textile-based electrodes, which can be used in the form of a wearable article.

WO-A-2004/097089 and DE-A-10 2006 058 765 disclose an electrically conductive elastic composite yarn. JP-A-1000 1851 and JP-A-2000 239 932 disclose an electroconductive spun yarn.

### BRIEF SUMMARY OF THE INVENTION

### Problem to be Solved by the Invention

However, a known electrode made of a conductive yarn can detect only one kind of biological information from detected signals; therefore, another electrode is required to measure other biological information such as respiration signals, etc., together with pulse signals.
However, this may cause discomfort during wear.

An object of the present invention is to provide a pressure-sensitive conductive yarn that is capable of detecting different biological information simultaneously when used as an electrode, and further a biological information-measuring garment including the pressure-sensitive conductive yarn.

### Means for solving the problem

The object of the present invention is achieved by a pressure-sensitive conductive yarn comprising a core yarn formed of an elastic yarn around which a winding yarn having conductivity is wound, wherein the winding yarn is formed by mixing a plurality of conductive fibers and nonconductive fibers, and spinning these fibers, to cause variations in electrical resistance with elongation or contraction.

In the pressure-sensitive conductive yarn, it is preferable that two winding yarns are wound around the core yarn, and that the winding direction of the first yarn is opposite to the winding direction of the second yarn.

The object of the present invention can be achieved by a biological information-measuring garment comprising electrodes that are arranged on the garment formed of a nonconductive material in such a manner as to closely contact with the body, wherein the electrodes are formed of the pressure-sensitive conductive yarn.

According to the biological information-measuring garment of the invention, a heart rate signal and respiration signal can be simultaneously extracted based on the output signals from the electrodes as different electrodes and a GND-electrode.

### Effect of the Invention

When the pressure-sensitive conductive yarn of the present invention is used as an electrode, different biological information can be detected at the same time.

### BRIEF DESCRIPTION OF SEVERAL VIEWS OF THE DRAWING

Fig. 1 is a schematic configuration view of one embodiment of the pressure-sensitive conductive yarn of the present invention.
Fig. 2 is a graph showing an example of the relationship between the tension (load) on the winding yarn, and the resistance value.
Fig. 3 is a configuration view of a measurement circuit used in the measurement shown in Fig. 2.
Fig. 4 is a graph showing an example of the relationship between the load on the winding yarn and the elongation rate of the winding yarn.
Fig. 5 is a graph showing the relationship between the elongation rate and the resistance value according to one embodiment of the pressure-sensitive conductive yarn.
Fig. 6 is a schematic configuration view of one embodiment of the biological information-measuring garment of the present invention.
Fig. 7 is a graph showing an example of the wave pattern of the output voltage detected from the biological information-measuring garment of Fig. 6.
Fig. 8(a) is a graph showing an example of the measurement results of heart rate signals. Fig. 8(b) is a graph showing an example of the reference signals of the heart rate signals.
Fig. 9(a) is a graph showing an example of respiration signals. Fig. 9(b) is a graph showing an example of the reference signals of the respiration signals.

### Explanation of Numerals

1: Pressure-sensitive conductive yarn
2: Core yarn
4, 6: Winding yarn
10: Biological information-measuring garment
12: Garment body
14,16: Different electrode
18: GND electrode

### DETAILED DESCRIPTION OF THE INVENTION

Embodiments of the present invention will now be described with reference to the accompanying drawings.

### Pressure-Sensitive Conductive Yarn

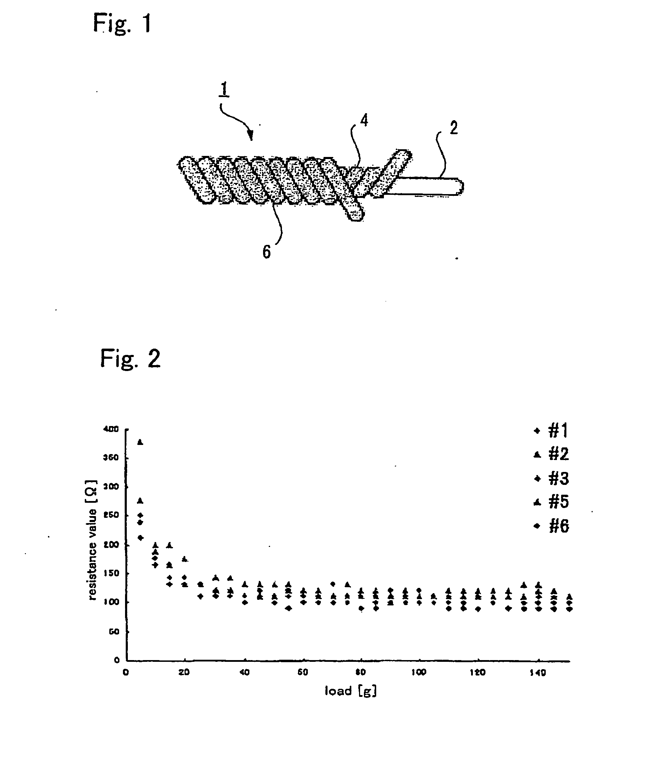

Fig. 1 is a schematic configuration view of one embodiment of the pressure-sensitive conductive yarn of the present invention. As shown in Fig. 1, the pressure-sensitive conductive yarn 1 is formed by doublewinding the winding yarn 4, 6 around the core yarn 2 composed of elastic yarn such as polyurethane. The winding direction of the first winding yarn 4 is opposite that of the second winding yarn 6. The pressure-sensitive conductive yarn 1 is produced by the same method as that of known double covering yarns.

The winding yarn 4, 6 is a mixed yarn of a plurality of conductive fibers such as stainless steel fibers and a plurality of nonconductive fibers such as polyester fibers; for example, those described in Japanese Unexamined Patent Publication No. 2003-20538 are preferably used. When a large amount of tension is exerted on the winding yarn 4, 6 having such properties, the density of the conductive fiber becomes large, resulting in low electrical resistance. In contrast, when a small amount of tension is exerted on the winding yarn, the density of the conductive fiber becomes small, resulting in high electrical resistance. That is, the electrical resistance value varies with changes in the tension on the winding yarn 4, 6.

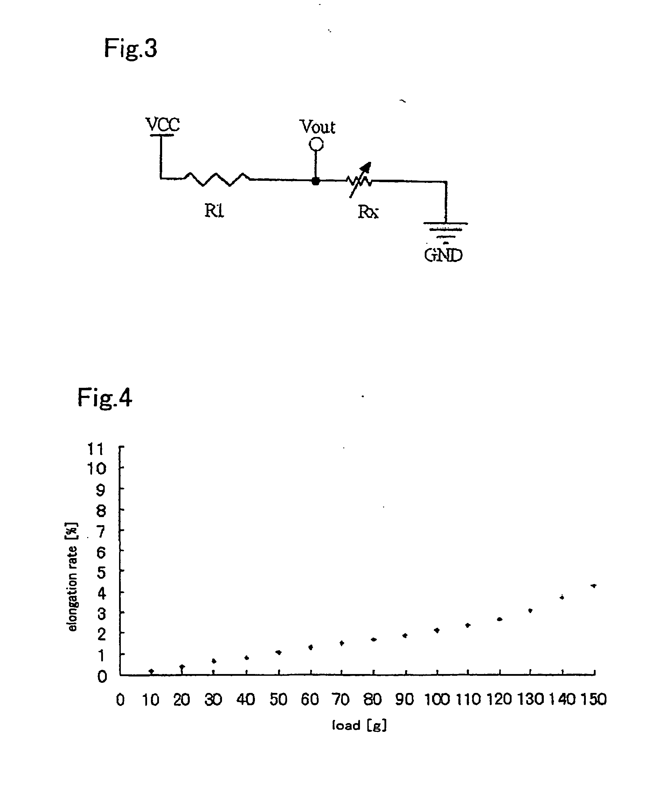

Fig. 2 is a graph showing an example of the relationship between the tension (load) on the winding yarn and the resistance value, and shows the measurement results of five winding yarns. As a winding yarn, a yarn having a blending ratio of 70/30 (polyester fiber/stainless fiber) was used. With the winding yarn (300 mm) having a weight at the end, changes in the resistance value of the winding yarn were measured using the measurement circuit of Fig. 3. In Fig. 3, VCC indicates a constant voltage (5V); R1, metal film resistor (1kΩ); and Rx, resistance of the subject winding yarn. The resistance value of Rx was calculated based on the output voltage (Vout) obtained when the VCC was divided by R1 and Rx. Water was used as a weight, and increased in 5 g increments up to 150 g. The measurement of "Vout" was conducted using a portable oscilloscope (ZR-MDR 10, produced by OMRON Corporation) at a sampling rate of 200 Hz.

As shown in Fig. 2, when the weight of the load is about 40 g or less, the resistance values of all of the winding yarns are significantly decreased as the load increases. This indicates that there is a correlation between the tension acting on the winding yarn and the resistance value. In contrast, when the weight of the load is 40 g or more, almost no change was observed in the resistance values of the winding yarns. This indicates that the measurement of the resistant value does not help to uniquely determine the weight of the load.

Fig. 4 is a graph showing an example of the relationship between the load acting on the winding yarn and the elongation rate of the winding yarn. The elongation rate is defined based on the natural length of the winding yarn. When the load has a good correlation with the resistance value, i.e., when the weight of the load is 40 g or less, the elongation rate is as small as 1% or less, as shown in Fig. 4. Therefore, when a winding yarn alone is used as an electrode, it is difficult to accurately extract biological signals that can be detected from body movements associated with respiration or the like.

On the other hand, in the pressure-sensitive conductive yarn 1 of the present embodiment, the winding yarn 4, 6 having the aforementioned properties is wound around the core yarn 2 composed of an elastic yarn. Therefore, even when the large amount of tension resulting from body movements acts on the pressure-sensitive conductive yarn 1 to greatly elongate the core yarn 2, the elongation of the winding yarn 4, 6 wound around the core yarn can be relatively suppressed. Accordingly, the entire elongation of the pressure-sensitive conductive yarn 1 can be detected as a small deformation of the winding yarn 4, 6, which allows for an accurate detection of biological signals associated with respiratory body movements or the like.

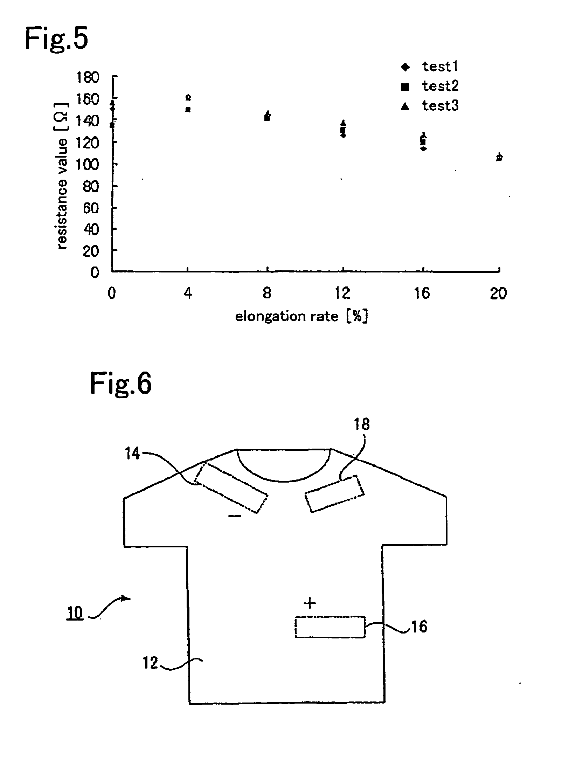

Fig. 5 is a graph showing an example of the relationship between the elongation rate and the resistance value according to the pressure-sensitive conductive yarn 1 of the present embodiment. The pressure-sensitive conductive yarn 1 had an initial length of 100 mm, and the elongation rate was measured up to 20% in 4% increments. The resistance value was measured in the same manner as in the measurement of the winding yarn alone described above, and the test was conducted three times for the same pressure-sensitive conductive yarn 1. As shown in Fig. 5, the resistance value was continuously changed until the elongation rate achieved 20%. This indicates that the pressure-sensitive conductive yarn of the present invention can detect a more significant elongation rate change as a resistance value change, compared to the results of the winding yarn alone, as shown in Figs. 2 and 4.

In the pressure-sensitive conductive yarn 1 of the present embodiment, the winding yarns 4,6, each having a different winding direction, are wound around the core yarn 2, which cancels out the torque, resulting in a stable yarn. Further, due to the variations in the contact density of the winding yarns 4, 6, the pressure-sensitive conductive yarn 1 of the present invention has a higher sensitivity than the pressure-sensitive conductive yarn having a single winding yarn. However, the winding yarn may be single wound around the core yarn. In this case also, biological signals associated with body movements can be detected by suppressing the elongation of the winding yarn relative to the core yarn.

Further, by employing the covering structure in which a material having stretch properties is used as the core yarn 2, the stretch properties of the core yarn 2 is stabilized, which reduces hysteresis and provides the winding yarn 4, 6 with a length enough for the entire deformation. Thereby, the winding yarn 4, 6 can be utilized in a stable deformation range, which allows for a stable detection compared to when the winding yarn 4, 6 is utilized alone without the core material 2.

### Biological information-measuring garment

The pressure-sensitive conductive yarn 1 as mentioned above is formed into a woven or knitted fabric to produce a sheet-like electrode. A biological information-measuring garment can be obtained by sewing the electrode on a garment.

Fig. 6 is a schematic configuration view of the biological information-measuring garment according to one embodiment of the present invention. The biological information-measuring garment 10 shown in Fig. 6 is used for measuring heart rate and respiration at the same time, and is provided with two different electrodes 14, 16 and a GND electrode (indifferent electrode) 18 inside the garment body 12. It is preferable that the garment body 12 be in the form of a T-shirt composed of a nonconductive material such as highly elastic polyurethane, and have a certain level of stretch properties so that the provided electrodes 14, 16, and 18 are easily attached to the body.

The different electrodes 14, 16 are provided on the right clavicular region and left subcostal region, respectively, according to the bipolar lead II of an ECG system, and the GND electrode 18 is provided on the left clavicular region. The different electrode 14 placed on the right clavicular region, different electrode 16 placed on the left subcostal region, and GND electrode 18 are all woven fabrics made of the pressure-sensitive conductive yarn 1 of Fig. 1, and are fixed to the garment body 12 by sewing or the like. The size of the different electrodes 14, 16 is, for example, about 60 × 30(mm).

To reduce the effects of noise from commercial power supplies, knitted fabrics or the like composed of the pressure-sensitive conductive yarn 1 may be provided as shields on the front side of the garment body 12 at the regions corresponding to the different electrodes 14 and 16. Further, to ensure the adhesion between the user's body and each of the different electrodes 14, 16, and GND electrode 18, an elastic body such as urethane foam may be inserted between the garment body 12 and each of the different electrodes 14, 16 and GND electrode 18. However, such shields and elastic bodies are not essential for the present invention.

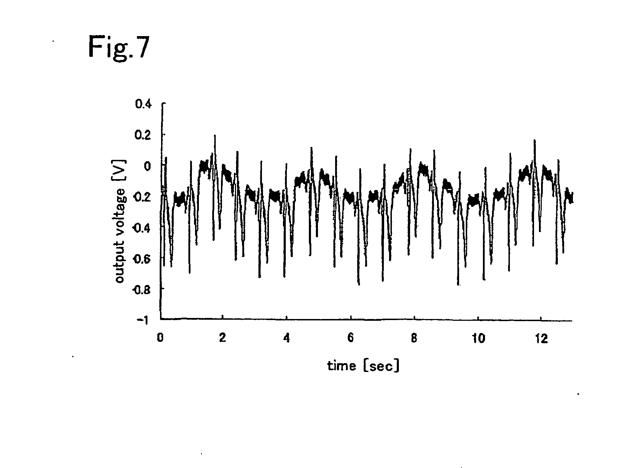

With the subject wearing the biological information-measuring garment 10 of Fig. 6, the output voltage, which is the potential difference of the different electrodes 14 and 16 based on the GND electrode 18, was measured. The gain was set to x510, and a primary high pass filter (HPF) having a cutoff frequency of 0.05 Hz and a fourth-order low pass filter (LPF) having a cutoff frequency of 30 Hz were used. An example of the output voltage is shown in Fig. 7. The output voltage was measured using a portable oscilloscope (ZR-MDR 10, produced by OMRON Corporation). It is possible to enter the value of the output voltage in the wristwatch-type information processing device to display and store it.

The output voltage shown in Fig. 7 includes a heart rate signal based on the electrocardiogram, as well as a respiration signal emitted from respiratory trunk movements. Specifically, when the elongation or contraction of the different electrode 16 on the left subcostal region causes a small deformation on the winding yarn 4, 6, the baseline oscillates due to the differential motion with the different electrode 14 on the right clavicular region. Using this information, respiration can be detected.

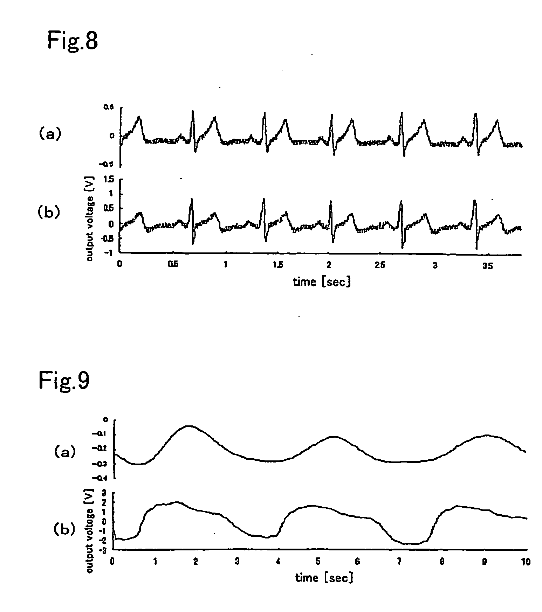

In conducting the measurement, heart rate signals were extracted by filtering the output voltage by HPF at 0.8 Hz while respiration signals were extracted by LPF at 0.8 Hz. The separated heart rate signals and respiration signals are shown in Fig. 8(a) and Fig. 9(a), respectively.

At the same time, the reference signals of each of the heart rate signals and respiration signals were measured. A disposable electrode (Blue Sensor, produced by Ambu) and a respiration pick-up (AP-C022, produced by Futami ME) were used for detecting the reference signals of the heart rate and that of the respiration, respectively. The signals are simultaneously measured and recorded using a Polymate (AP1524, produced by TECH). Fig. 8(b) and Fig. 9(b) show the heart rate reference signals and respiration reference signals, respectively.

The comparison between Figs. 8(a) and (b), and the comparison between Figs. 9(a) and (b) reveal that both of the heart rate signals and respiration signals have wave patterns similar to those of the reference signals, indicating that the biological information-measuring garment 10 of the present embodiment can detect a heart rate signal and respiration signal at the same time.

In the biological information-measuring garment 10 of the present embodiment, the location and the number of electrodes are not particularly limited, and can be suitably changed depending on the subject biological information and measurement principal, such as electromyography and brain waves. Further, the form of the garment body 12 on which the electrodes are attached is not limited to T-shirts, and any garment or clothing accessory can be selected in accordance with the location of the electrodes. In the present embodiment, both electrodes are formed using the pressure-sensitive conductive yarn of the present invention; however, only one side of the electrodes, i.e., the electrode placed on the region elongated and contracted by body movements, may be formed of the pressure-sensitive conductive yarn of the present invention.

In addition to the known biological signal measurements using an electrode, the biological information-measuring garment of the present invention can simultaneously detect biological signals that result from body movements, such as respiration, movement of the shoulder joints, leaning of the trunk, movement of the neck by feeling of fullness or swallowing, etc. Therefore, the present invention is particularly appropriate for this purpose.

## Claims

1. A pressure-sensitive conductive yarn (1) comprising a core yarn (2) formed of an elastic yarn around which a winding yarn (4, 6) having conductivity is wound, **characterised in that** the winding yarn (4, 6) is formed by mixing a plurality of conductive fibers and a plurality of nonconductive fibers, and spinning these fibers, to cause variations in its electrical resistance with elongation or contraction.

2. The pressure-sensitive conductive yarn (1) according to claim 1, wherein two winding yarns are wound around the core yarn (2) the winding direction of the first yarn (4) being opposite to the winding direction of the second yarn (6).

3. A biological information-measuring garment (10) comprising electrodes (14, 16, 18) provided on the garment formed of a nonconductive material in such a manner as to closely contact with the body, wherein the electrodes (14, 16, 18) are formed using the pressure-sensitive conductive yarn according to claim 1 or 2.

4. The biological information-measuring garment (10) according to claim 3, comprising two electrodes (14, 16) as different electrodes and a GND-electrode (18), wherein a heart rate signal and respiration signal can be simultaneously extracted based on an output signal, which is a potential difference of the two different electrodes (14, 16) based on the GND-electrode (18).

## Patentansprüche

1. Druckempfindliches leitfähiges Garn (1), umfassend ein Kerngarn (2), gebildet aus einem elastischen Garn, um welches ein Wickelgarn (4,6) mit Leitfähigkeit gewickelt ist, **dadurch gekennzeichnet, dass** das Wickelgarn (4,6) durch Mischen einer Vielzahl von leitfähigen Fasern und einer Vielzahl von nichtleitfähigen Fasern, und dem Spinnen dieser Fasern gebildet ist, um Variationen in seinem elektrischen Widerstand mit Ausdehnung oder Kontraktion zu bewirken.

2. Druckempfindliches leitfähiges Garn (1) nach Anspruch 1, wobei zwei Wickelgarne um das Kerngarn (2) gewickelt sind, wobei die Wickelrichtung des ersten Garns (4) entgegengesetzt der Wickelrichtung des zweiten Garns (6) ist.

3. Kleidungsstück (10) zur Messung biologischer Informationen, umfassend Elektroden (14,16,18), bereitgestellt auf dem Kleidungsstück, gebildet aus einem nicht-leitenden Material in einer solchen Weise, um in engem Kontakt mit dem Körper zu sein, wobei die Elektroden (14,16,18) unter Verwendung des druckempfindlichen leitfähigen Garns nach Anspruch 1 oder 2 gebildet sind.

4. Kleidungsstück (10) zur Messung biologischer Informationen nach Anspruch 3, umfassend zwei Elektroden (14,16) als verschiedene Elektroden und eine Masseelektrode bzw. GND-Elektrode (18), wobei ein Herzfrequenzsignal und ein Atmungssignal gleichzeitig extrahiert werden können, basierend auf einem Ausgabesignal, welches eine Potentialdifferenz der zwei verschiedenen Elektroden (14, 16), bezogen auf die Masseelektrode (18), ist.

## Revendications

1. Fil conducteur sensible à la pression (1) comprenant un fil d'âme (2) formé d'un fil élastique autour duquel est enroulé un fil d'enroulement (4, 6) ayant une conductivité, **caractérisé en ce que** le fil d'enroulement (4, 6) est formé en mélangeant une pluralité de fibres conductrices et une pluralité de fibres non conductrices, et en filant ces fibres, afin de provoquer des variations de sa résistance électrique par allongement ou contraction.

2. Fil conducteur sensible à la pression (1) selon la revendication 1, dans lequel deux fils d'enroulement sont enroulés autour du fil d'âme (2), la direction d'enroulement du premier fil (4) étant opposée à la direction d'enroulement du second fil (6).

3. Vêtement de mesure d'informations biologiques (10) comprenant des électrodes (14, 16, 18) disposées sur le vêtement formé d'un matériau non conducteur de façon à être en contact étroit avec le corps, dans lequel les électrodes (14, 16, 18) sont formées à l'aide du fil conducteur sensible à la pression selon la revendication 1 ou 2.

4. Vêtement de mesure d'informations biologiques (10) selon la revendication 3, comprenant deux électrodes (14, 16) en tant qu'électrodes différentes et une électrode de masse (18), dans lequel un signal de fréquence cardiaque et un signal de respiration peuvent être simultanément extraits d'après un signal de sortie, qui est une différence de potentiel des deux électrodes (14, 16) différentes d'après l'électrode de masse (18).
